# EUROPEAN PATENT APPLICATION

(11) **EP 4 628 791 A1**
(43) Date of publication of application: **08.10.2025**
(21) Application number: 23896948.9
(22) Date of filing: 01.12.2023
(51) Int. Cl.: F23L 7/00, F27D 17/00, F27B 17/00

(54) **METHOD FOR REDUCING NITROGEN OXIDE EMISSION AND COMBUSTION SYSTEM**

(30) Priority: 02.12.2022 CN 202211537550; 02.12.2022 CN 202211537533
(71) Applicant: China Petroleum & Chemical Corporation, Beijing 100728 (CN); Sinopec Engineering Incorporation, Beijing 100101 (CN)
(72) Inventor: HE, Xiou, Beijing 100101 (CN); LI, Changli, Beijing 100101 (CN); WANG, Zizong, Beijing 100728 (CN); XIAO, Jia, Beijing 100101 (CN); WANG, Meng, Beijing 100101 (CN); LIN, Jiangfeng, Beijing 100101 (CN); BAI, Fei, Beijing 100101 (CN); SHI, Yu, Beijing 100101 (CN); XUE, Lei, Beijing 100101 (CN)
(74) Representative: Petraz, Gilberto Luigi
(86) International application number: PCT/CN2023/135938
(87) International publication number: WO 2024/114806

(57) **Abstract**

The present invention relates to the field of flue gas nitrogen oxide emission, and in particular to a method and a combustion system for reducing nitrogen oxide emission. Introducing non-nitrogen inert gas as a combustion diluent to establish ignition; when the amount of circulation flue gas reaches a designed value, the introduction of the non-nitrogen inert gaseous diluent is stopped; and by means of circulation of a part of flue gas and a part of CO₂ in the flue gas, the internal circulation of the whole process system is ensured. The present invention achieves the objectives of reducing the nitrogen oxide emission and saving the fuel, thereby saving the energy, protecting the environment, fundamentally solving the emission problem of NOx in the flue gas, and achieving carbon reduction.

## Description

### Cross-Reference to Related Application

The present application claims the priority of Chinese patent application No. 202211537533.4 entitled "Method for reducing nitrogen oxide emission in ethylene cracking furnace" and filed on December 2, 2022, the content of which is incorporated herein by reference in its entirety.

The present application claims the priority of Chinese patent application No. 202211537550.8 entitled "Method for reducing nitrogen oxide emission in refinery heating furnace" and filed on December 2, 2022, the content of which is incorporated herein by reference in its entirety.

### Field of the Invention

The present invention belongs to the field of nitrogen oxide emission in flue gas, and specifically to a method for reducing nitrogen oxide emission and a combustion system.

### Background of the Invention

Tubular heating furnace is a process heating furnace used in petroleum refining industries, petrochemical and chemical industries, and chemical fiber industries. The ethylene cracking furnace, a critical component of ethylene production plants, processes various raw materials such as natural gas, refinery gas, crude oil, naphtha into olefin-rich cracking gas. This gas is subsequently delivered to downstream units for conversion into ethylene, propylene, and other by-products. The tubular heating furnace or the ethylene cracking furnace is generally provided with a radiation section and a convection section, where the process fluids flow from top to bottom. The in-tube medium of the radiation section is subjected to radiative heat transfer through flames or a high-temperature flue gas, while the in-tube medium of the convection section undergoes convective heat transfer through flue gas exiting the radiation section. High-temperature flue gas generated by combustion in the furnace passes through the radiation section and convection section, and then enters a waste heat recovery system to exchange heat with combustion air, so as to further improve thermal efficiency. After heat exchange, the flue gas is directly discharged through a chimney via an induced draft fan. To comply with environmental regulations, reducing nitrogen oxide (NOx) emission from the tubular heating furnace such as a refinery heating furnace and the ethylene cracking furnace is imperative.

The NOx generated by combustion mainly comprises NO and NO₂, with NO accounting for 90-95%. NOx formation mechanisms are classified into three types: a thermal type, a rapid type, and a fuel type. In practical operations, NOx emissions from ethylene cracking furnace or the refinery heating furnace is mainly derived from thermal-type NOx. The thermal-type NOx generally refers to the NOx generated by a chain oxidation reaction of nitrogen gas and oxygen gas in a high temperature zone in the presence of excessive oxygen gas during the combustion of fuel and air. Thermal-type NOx mainly occurs in a local high-temperature zone formed by uneven heat distribution in the furnace. Residence time of the flue gas in high-temperature zone also has an impact on the generated amount of the NOx levels, the longer the residence time, the higher the generated amount of NOx will be.

At present, two types of technologies are used globally to reduce NOx emissions from the tubular heating furnace or the ethylene cracking furnace: one is low-NOx combustion technology; the other is post-combustion treatment, i.e. flue gas denitrification technology to reduce NOx concentrations in exhaust gases. Both types of technologies have been applied in industries, achieving different denitrification effects as required.

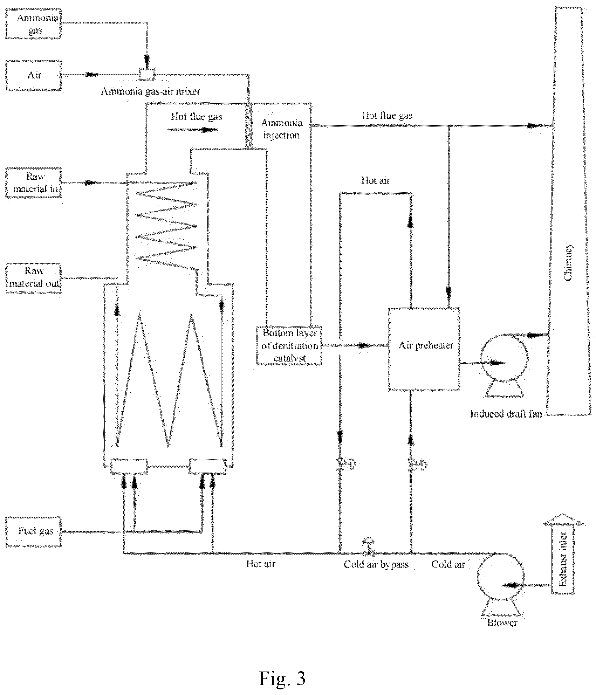

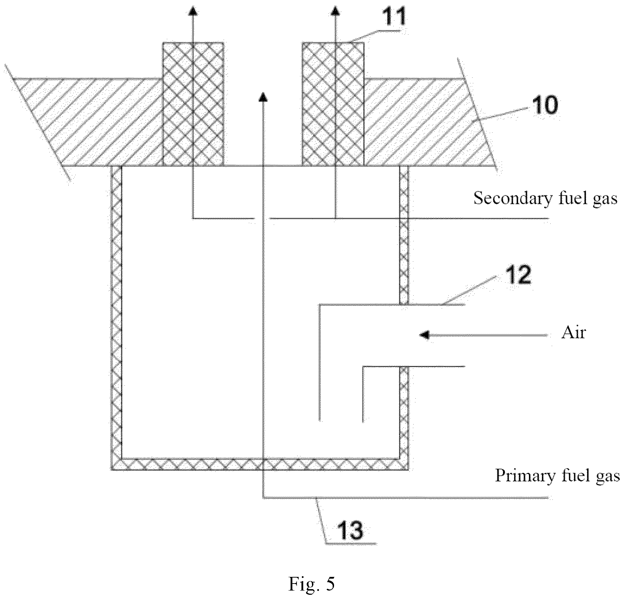

The low-NOx combustion technologies mainly used in a burner of the refinery heating furnace or the ethylene cracking furnace comprise: air staging, fuel staging, internal flue gas recirculation, external flue gas recirculation, etc., which suppress the generation of the NOx by reducing core combustion temperature (eliminating localized hot spots), and shortening flue gas residence time in high-temperature zone, etc. The currently-adopted low-NOx burner usually uses a combination of the fuel classification and the flue gas recirculation technology, which can effectively reduce the temperature at the center of the flame, thereby lowering the NOx concentration in the flue gas. Meanwhile, the temperature at the center of the burner flame may be reduced by injecting superheated steam through a steam spray gun located in the burner, so as to suppress the generation of thermal-type NOx (as shown in Figures 3 and 5). At present, such a technology is commonly used, and the NOx emission concentration in the flue gas may be reduced to 70 mg/Nm³ or less, but an operating cost of this technology is high.

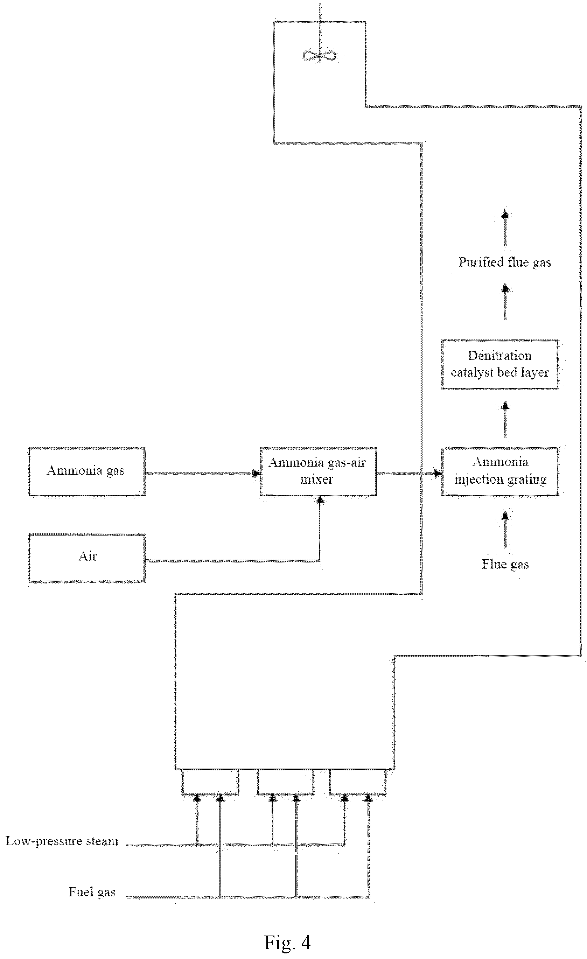

Selective catalytic reduction (SCR) technology is currently the most widely used flue gas denitrification technology in industrial applications. In heating furnace or ethylene cracking furnace, SCR reactors typically installed in the middle of the convection section, between convection section and air preheater, or between air preheaters. The position of the SCR reactor may be selected according to the flue gas temperature and site conditions. In a reactor, reductant is sprayed into the flue gas with a temperature of about 280-420°C to catalytically convert NOx to N₂ and H₂O, thereby significantly reducing the emission concentration of the NOx in the flue gas (as shown in Figure 4). However, since an additional layout space for SCR catalysts is needed, it will lead to an increase in the power of the induced draft fan and an increase in the suction force of the chimney; and the replacement of catalysts at the end of their lifespan, the use of the reducing agent, and the increase in the power of the fan all have a relatively significant impact on the fixed cost and operating cost of the apparatus.

Chinese patent CN207585344U discloses an energy-saving and emission reducing system for the tubular heating furnace, in which air and circulation flue gas are adopted as combustion adjuvants, and a circulation amount of flue gas is 30%; and the system can save apparatus investment by 40%. How to effectively utilize more circulation flue gas in a more energy-saving and environment-friendly manner is a key factor to consider in energy conservation. In addition, since the air is adopted, this patent does not disclose how to reduce nitrogen oxide emission in the heating furnace.

### Summary of the Invention

Therefore, in view of the problem of high cost for reducing nitrogen oxide emission within industrial furnaces, the objective of the present invention is to provide a method and a combustion system for minimizing nitrogen oxide emission. The method involves introducing non-nitrogen inert gaseous diluent to replace gas inside furnace; mixing non-nitrogen inert gaseous diluent and oxygen gas, introducing the mixed gas into the firebox to be mixed with fuel, and then igniting the mixture to initiate combustion; and mixing a part of the cooled flue gas and the remaining flue gas from the flue gas outlet with oxygen gas, then introducing the obtained mixture into the furnace to be mixed with a fuel for combustion. This integrated approach establishes a nitrogen-lean environment, the generated flue gas does not contain NOx, thereby fundamentally solving the problem of NOx emission in the flue gas.

The objective of the present application is achieved by the following technical solutions.

In a first aspect, the present invention provides a method for reducing nitrogen oxide emission, which comprises reducing N₂ content in a system by the following manners:
I. introducing a non-nitrogen inert gaseous diluent to replace gas inside a furnace;
II. mixing non-nitrogen inert gaseous diluent and oxygen gas, introducing the mixed gas into the furnace to be mixed with a fuel, and then igniting the mixture to initiate combustion; preferably, the mixed gas is introduced into the burner of the furnace; and/or
III. cooling at least a part of flue gas from the flue gas outlet to obtain a cooled flue gas, mixing a part of the cooled flue gas and remaining flue gas from the flue gas outlet with oxygen gas, or mixing all the flue gas from the flue gas outlet with oxygen gas, then introducing the obtained mixture into the furnace to be mixed with fuel for combustion; preferably, the obtained mixture is introduced into the burner of the furnace;
wherein, the non-nitrogen inert gaseous diluent is selected from one or two of CO₂ gas and superheated steam.

Preferably, in manner I, the determination of nitrogen in the gas at an outlet of the furnace complies with NOx emission. Oxygen gas and hydrocarbons meet the explosion-proof requirements, and then the replacement is completed; and/or
in manner II, a condition for ignition is as follows: a volume ratio of the oxygen gas in the mixed gas of the non-nitrogen inert gaseous diluent and the oxygen gas reaches 10-30%; and/or
in manner III, a volume ratio of the flue gas which is subjected to cooling to all the flue gas is 25-100%; preferably, the volume ratio is 70-100%; more specifically, the volume ratio is: 75%, 80%, 85%, 90%, 95%; and/or
a temperature of the superheated steam as the non-nitrogen inert gaseous diluent is 120-300°C, a low pressure steam is preferably used, while a medium pressure steam is used as an alternative; according to some specific embodiments, the temperature of the superheated steam is 120°C, 140°C, 160°C, 180°C, 190°C, 200°C, 210°C, 230°C, 250°C, 280°C; and/or
the fuel is a fuel gas and/or a fuel oil with low nitrogen content, preferably a mixture of methane and hydrogen gas, or methane; a ratio of methane in the mixture of methane and hydrogen gas is preferably 5-95 mol%; according to some specific embodiments, the ratio of methane in the mixture of methane and hydrogen is 10 mol%, 20 mol%, 40 mol%, 60 mol%, 65 mol%, 70 mol%, 80 mol%, 90 mol%.

Furthermore, manner III includes:
feeding at least a part of the flue gas from a the flue gas outlet into a heat source inlet of a first heat exchanger and cooling it in the first heat exchanger, to obtain the cooled flue gas at a heat source outlet of the first heat exchanger; and feeding a part of the cooled flue gas and the remaining flue gas from the flue gas outlet into the furnace, preferably into the burner of the furnace; and
preferably, the cooled flue gas is carbon dioxide, and more preferably, a remaining cooled flue gas is fed to a carbon dioxide capture unit for collection.

Furthermore, a volume ratio of the cooled flue gas fed into the carbon dioxide capture unit to the cooled flue gas from the heat source outlet of the first heat exchanger is 9-21:100;
according to some specific embodiments, the volume ratio of the cooled flue gas fed into the carbon dioxide capture unit to the cooled flue gas from the heat source outlet of the first heat exchanger is 10%, 12%, 15%, 16%, 18%, and 20%; and/or
the first heat exchanger comprises one or more heat exchange units connected in series, with heat fluid being the flue gas, and with cold fluid being independently O₂, the fuel, the cooled flue gas to be fed into the burner or a raw material to be treated, and the first heat exchanger is used for heating the O₂, the fuel, the cooled flue gas to be fed into the burner or the raw material to be treated; and/or
a second heat exchanger is further included, with heat fluid being a liquid at the heat source outlet of the first heat exchanger, and cold fluid being independently O₂, a fuel or a raw material, and is used for preheating the O₂, the fuel or the raw material; and
preferably, the O₂ and/or the fuel to be fed into the burner are firstly fed into a cold source inlet of the second heat exchanger for preheating; and the preheated O₂ and/or fuel are then fed into cold source inlets of individual heat exchange units of the first heat exchanger for heating.

Furthermore, the method specifically includes the following steps:
optionally, (1) filling the non-nitrogen inert gaseous diluent into the furnace through a non-nitrogen inert gaseous diluent pipeline, and discharging it through an outlet of a convection section and/or an exhaust pipeline on a circulating flue gas pipeline to replace gas inside the furnace;
(2) injecting oxygen gas into a circulation main pipeline or the circulation flue gas pipeline through an oxygen gas pipeline; injecting the non-nitrogen inert gaseous diluent into the circulation main pipeline or the circulation flue gas pipeline through the non-nitrogen inert gaseous diluent pipeline; allowing the flue gas from the flue gas outlet or an outlet of the convection section to pass through the circulation flue gas pipeline and the circulation main pipeline in sequence, then introducing it into a bottom of the furnace; after a predetermined oxygen gas content is reached, introducing fuel into the furnace through a fuel pipeline and igniting the mixture to initiate combustion; and stopping the introduction of the non-nitrogen inert gaseous diluents until an amount of circulation flue gas reaches a designed minimum required value; and
(3) cooling at least a part of the flue gas from the flue gas outlet to obtain the cooled flue gas, mixing in the circulation main pipeline a part of the cooled flue gas and the remaining flue gas from the flue gas outlet with oxygen gas from the oxygen gas pipeline, or mixing all the flue gas from the flue gas outlet with oxygen gas, introducing the obtained mixture into the furnace, then introducing fuel into the furnace through the fuel pipeline, to allow continuous combustion.

Furthermore,
an amount of the circulation flue gas reaches the designed minimum required value, which is an amount of flue gas as a diluent required during normal operation of the furnace at a lowest load; and/or
the remaining cooled flue gas of step (3) is fed to a carbon dioxide capture unit.

Optionally, manner I may further comprise replacing gas inside the furnace by adopting the following steps:
S1. introducing air through an air inlet located on the circulation flue gas pipeline or the circulation main pipeline, and after analysis in the furnace is qualified, introducing fuel for ignition;
S2. when the flue gas temperature leaving a radiation section is higher than the non-nitrogen inert gaseous diluent temperature, introducing the non-nitrogen inert gaseous diluent to replace gas inside the furnace; and gradually stopping the introduction of the air while increasing flow rate of the diluent.

In order to prevent the temperature at the radiation section and the convection section from being lower than a dew point temperature of the non-nitrogen inert gaseous diluent after the non-nitrogen inert gaseous diluent is injected, it is necessary to define that the temperature of the flue gas leaving the radiation section is higher than that of the non-nitrogen inert gaseous diluent, so as to avoid unfavorable combustion or damage to the substrate and/or the furnace tube.

Another objective of the present invention is to protect a combustion system with low nitrogen oxide emission, which comprises a furnace, a flue gas circulation system, an oxygen gas pipeline, a fuel pipeline, and a control system;
the furnace is sequentially provided with a radiation section and a convection section from bottom to top; and radiation and convection correspond to two ways of heat transfer, with the radiation section having a relatively high temperature and the convection section having a relatively low temperature, wherein the radiation section relies on thermal radiation to transfer heat, while the convection section mainly relies on convection to transfer heat, so as to heat the medium;
the radiation section is provided therein with a burner; a flue gas outlet is used for emission of flue gas after combustion;
the flue gas circulation system comprises a circulation flue gas pipeline and a circulation main pipeline; two ends of the circulation flue gas pipeline are respectively connected to the flue gas outlet and the circulation main pipeline respectively, and are used for feeding the flue gas from the flue gas outlet, i.e. the circulation flue gas, into the circulation main pipeline, to achieve the circulation of flue gas;
the oxygen gas pipeline is connected to the circulation main pipeline or the circulation flue gas pipeline, and is used for feeding oxygen gas into the circulation main pipeline;
the circulation main pipeline is connected to the burner via a branch pipe and is used for feeding a mixture of a circulation flue gas or a diluent and oxygen gas into the firebox;
the fuel pipeline is connected to the burner, and is used for feeding a fuel into the burner; and
a non-nitrogen inert gaseous diluent pipeline is further included, and the non-nitrogen inert gaseous diluent pipeline is connected to the circulation flue gas pipeline or the circulation main pipeline.

Preferably, the flue gas circulation system further comprises a circulation carbon dioxide pipeline; the circulation carbon dioxide pipeline is connected to the outlet of the convection section and the circulation flue gas pipeline; the combustion system further comprises a flue gas heat exchange system located between the circulation carbon dioxide pipeline and the outlet of the convection section, wherein the flue gas heat exchange system comprises a first heat exchanger for partially or completely removing moisture from the flue gas; and/or
the outlet of the convection section is connected to a heat source inlet of the first heat exchanger, a heat source outlet of the first heat exchanger is connected to an inlet of the circulation carbon dioxide pipeline; an outlet of the circulation carbon dioxide pipeline is connected to the circulation flue gas pipeline or the circulation main pipeline and is used for feeding a cooled flue gas into the circulation flue gas pipeline or the circulation main pipeline, and in turn into the firebox, and an inlet of the carbon dioxide capture unit is connected to the heat source outlet of the first heat exchanger.

Furthermore, the first heat exchanger comprises one or more heat exchange units connected in series; wherein heat fluid is the flue gas, and cold fluid is independently O₂ to be fed into the furnace, a fuel, a circulation CO₂ or a raw material to be treated, and the first heat exchanger is used for heating the O₂, the fuel, the circulation CO₂ or the raw material to be treated; preferably, the first heat exchanger comprises 2-4 heat exchange units connected in series, wherein a heat source inlet of a first heat exchange unit is connected to the outlet of the convection section; a heat source outlet of each heat exchange unit is connected to a condensate water pipeline, and a heat source outlet of the last heat exchanger is connected to the circulation carbon dioxide pipeline;
more preferably, the first heat exchanger comprises a heat exchange unit H1-1, a heat exchange unit H1-2, a heat exchange unit H1-3, and a heat exchange unit H1-4 connected in series, wherein a cold source inlet of the heat exchange unit H1-1 is connected to a raw material pipeline, a cold source outlet thereof is connected to the convection section of the furnace, a heat source inlet thereof is connected to the outlet of the convection section, and a heat source outlet thereof is connected to the condensate water pipeline and a heat source inlet of heat exchange unit H1-2;
a cold source inlet of the heat exchange unit H1-2 is connected to the fuel pipeline, a cold source outlet thereof is connected to the burner, and a heat source outlet thereof is connected to the condensate water pipeline and a heat source inlet of heat exchange unit H1-3;
a cold source inlet of the heat exchange unit H1-3 is connected to the oxygen gas pipeline, a cold source outlet thereof is connected to the circulation main pipeline, and a heat source outlet thereof is connected to the condensate water pipeline and a heat source inlet of heat exchange unit H1-4; and
a cold source inlet of the heat exchange unit H1-4 is connected to a partial circulation carbon dioxide pipeline, a cold source outlet thereof is connected to the circulation carbon dioxide pipeline, for heating gas in the partial circulation carbon dioxide pipeline; and a heat source outlet thereof is connected to the condensate water pipeline and the circulation carbon dioxide pipeline.

Furthermore, the flue gas heat exchange system further comprises a second heat exchanger located between the first heat exchanger and the condensate water pipeline, that is, a heat source inlet of the second heat exchanger is connected to the heat source outlet of the first heat exchanger; and/or
the second heat exchanger comprises at least 2 heat exchange units connected in series;
more preferably, a cold source inlet of the heat exchange unit H2-1 is connected to the fuel pipeline, a cold source outlet thereof is connected to the cold source inlet of the heat exchange unit H1-2 of the first heat exchanger or the burner, a heat source inlet thereof is connected to the heat source outlet of the first heat exchanger, and a heat source outlet thereof is connected to a heat source inlet of heat exchange unit H2-2; and/or
a cold source inlet of the heat exchange unit H2-2 is connected to the oxygen gas pipeline, a cold source outlet thereof is connected to the circulation main pipeline, a heat source inlet is connected to the heat source outlet of the heat exchange unit H2-1, and a heat source outlet thereof is connected to the condensate water pipeline.

Furthermore, the control system comprises: control valves located on the circulation flue gas pipeline, the diluent pipeline, the oxygen gas pipeline, the fuel pipeline respectively, to control an amount of gas; and a control valve located on the circulation carbon dioxide pipeline to control an amount of carbon dioxide in the circulation carbon dioxide pipeline.

The furnace is a fire heating furnace, preferably, a refinery heating furnace or an ethylene cracking furnace.

The advantageous effects of the present invention are as follows:
1. By utilizing the mixture of the non-nitrogen inert gas such as CO₂ and/or superheated steam with oxygen gas as the combustion medium, it is ensured that no NOx is generated during combustion, thereby fundamentally eliminating NOx formation during combustion;
2. When a mixture of CO₂ and/or superheated steam as diluent with oxygen gas is used instead of air to provide the oxygen gas required for fuel combustion, the main combustion products are CO₂ and water; and the latent heat of superheated steam is used for cooling while separating CO₂, such that the capturing and recycling of CO₂ can be achieved at low cost.
3. At steady state, there is no need to additionally supplement the non-nitrogen inert gaseous diluent; the circulation of the entire process is achieved through partial circulation of CO₂, achieving closed-loop operation through a simple flow control. Meanwhile, sensible heat and the latent heat recovery of the flue gas is achieved through heat exchanging system, thereby saving energy and saving fuel consumption by 1.5% to 15% while ensuring normal combustion.

### Brief Description of the Drawings

By means of the exemplary embodiments of the present invention which are described in more detail in combination with the accompanying drawings, the above and other objectives, features and advantages of the present invention will become more apparent. Among others, in the exemplary embodiments of the present invention, the same reference numerals typically represent the same components.

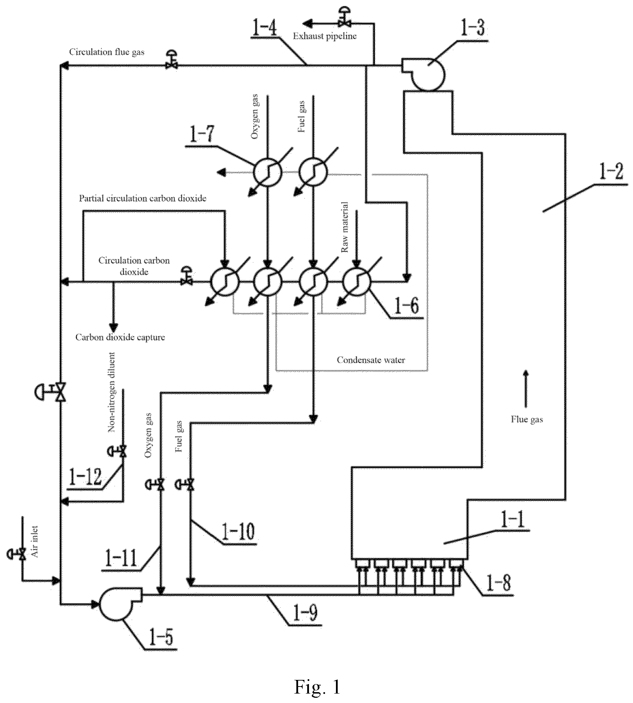

Figure 1 shows a schematic diagram of the implementation of the present invention for reducing NOx emission in an ethylene cracking furnace by using a mixture of non-nitrogen inert gaseous diluent with oxygen gas as a combustion adjuvant;

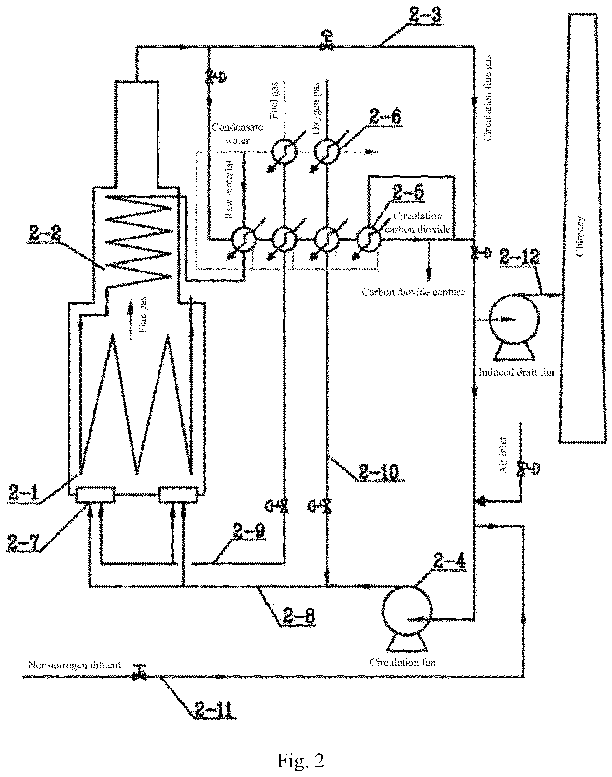

Figure 2 shows a schematic diagram of the implementation of the present invention for reducing NOx emission in a refinery heating furnace by using a mixture of non-nitrogen inert gaseous diluent with oxygen gas as a combustion adjuvant;
Figure 3 shows a schematic diagram of an apparatus and a flow process for reducing NOx emission in a heating furnace by applying an existing technology;
Figure 4 shows a schematic diagram of an apparatus and a flow process for reducing NOx emission in an ethylene cracking furnace by applying an existing SCR technology; and
Figure 5 shows a schematic diagram of an apparatus and a flow process for low-NOx burner used in an existing ethylene cracking furnace.

### Description of reference numerals

1-1. Radiation section of cracking furnace; 1-2. Convection section of cracking furnace; 1-3. Induced draft fan of cracking furnace; 1-4 Circulation flue gas pipeline of cracking furnace; 1-5. Circulation fan of cracking furnace; 1-6. First heat exchanger of cracking furnace; 1-7. Second heat exchanger of cracking furnace; 1-8. Burner of cracking furnace; 1-9. Circulation main pipeline of cracking furnace; 1-10. Fuel pipeline of cracking furnace; 1-11. Oxygen gas pipeline of cracking furnace; 1-12. Non-nitrogen inert gaseous diluent pipeline of cracking furnace;
2-1. Radiation section of heating furnace; 2-2. Convection section of heating furnace; 2-3. Circulation flue gas pipeline of heating furnace; 2-4. Circulation fan of heating furnace; 2-5. First heat exchanger of heating furnace; 2-6. Second heat exchanger of heating furnace; 2-7. Burner of heating furnace; 2-8. Circulation main pipeline of heating furnace; 2-9. Fuel pipeline of heating furnace; 2-10. Oxygen gas pipeline of heating furnace; 2-11. Non-nitrogen inert gaseous diluent pipeline of heating furnace; 2-12. Bypass flue of heating furnace;
10. Burner; 11. Burner brick; 12. Inlet duct of burner; 13. Fuel pipeline.

### Detailed Description of the Embodiments

Hereafter, the embodiments of the present invention will be described in detail in combination with the examples, but those skilled in the art will understand that the following examples are only used to illustrate the present invention, but should not be considered to limit the scope of the present invention.

A combustion system of the present application comprises a furnace, a flue gas circulation system, a flue gas heat exchange system, an oxygen gas pipeline, a fuel pipeline, and a control system;
the flue gas heat exchange system comprises a first heat exchanger and a carbon dioxide capture unit;
the furnace is provided with a radiation section and a convection section in sequence from bottom to top; the radiation section is provided therein with a burner; a flue gas outlet is used for flue gas emission after combustion; the flue gas circulation system comprises a circulation flue gas pipeline, a circulation carbon dioxide pipeline, and a circulation main pipeline; an inlet of the circulation flue gas pipeline is connected to an outlet of the convection section, and an outlet of the circulation flue gas pipeline is connected to an burner through the circulation main pipeline; an inlet of the circulation carbon dioxide pipeline is connected to a heat source outlet of the first heat exchanger, and an outlet of the circulation carbon dioxide pipeline is connected to the circulation flue gas pipeline and is used for feeding cooled flue gas into the circulation flue gas pipeline; and the circulation carbon dioxide pipeline is further connected to the carbon dioxide capture unit;
the oxygen gas pipeline is connected to the circulation main pipeline and is used for feeding oxygen gas into the circulation main pipeline; and the fuel pipeline is connected to the burner of the furnace and is used for feeding fuel into the furnace;
the first heat exchanger comprises one or more heat exchange units connected in series, with heat fluid being the flue gas, and cold fluid being independently O₂ to be fed into the furnace, a fuel, a circulation CO₂ or a raw material to be treated, and the first heat exchanger is used for heating the O₂, the fuel, the circulation CO₂ or the raw material to be treated; preferably, the first heat exchanger comprises 2-4 heat exchange units connected in series, wherein a heat source inlet of a first heat exchange unit is connected to the outlet of the convection section; a heat source outlet of each heat exchange unit is connected to a condensate water pipeline, and a heat source outlet of the last heat exchanger is connected to the circulation carbon dioxide pipeline;
more preferably, the first heat exchanger comprises a heat exchange unit H1-1, a heat exchange unit H1-2, a heat exchange unit H1-3, and a heat exchange unit H1-4 connected in series, wherein a cold source inlet of the heat exchange unit H1-1 is connected to a raw material pipeline, a cold source outlet thereof is connected to the convection section of the furnace, a heat source inlet thereof is connected to the outlet of the convection section, and a heat source outlet thereof is connected to the condensate water pipeline and a heat source inlet of heat exchange unit H1-2;
more specifically, the cold source inlet of the heat exchange unit H1-1 is connected to the raw material pipeline, the cold source outlet thereof is connected to the convection section of the furnace, the heat source inlet thereof is connected to the outlet of the convection section of the furnace, and the heat source outlet thereof is connected to the condensate water pipeline and the heat source inlet of the heat exchange unit H1-2;
a cold source inlet of the heat exchange unit H1-2 is connected to the fuel pipeline, a cold source outlet thereof is connected to the burner, a heat source inlet thereof is connected to the heat source outlet of the heat exchange unit H1-1, and a heat source outlet thereof is connected to the condensate water pipeline and a heat source inlet of heat exchange unit H1-3;
a cold source inlet of the heat exchange unit H1-3 is connected to the oxygen gas pipeline, a cold source outlet thereof is connected to the circulation main pipeline, a heat source inlet thereof is connected to the heat source outlet of the heat exchange unit H1-2, and a heat source outlet thereof is connected to the condensate water pipeline and a heat source inlet of heat exchange unit H1-4;
a cold source inlet of the heat exchange unit H1-4 is connected to a partial circulation carbon dioxide pipeline, a cold source outlet thereof is connected to the circulation carbon dioxide pipeline, for heating gas in the partial circulation carbon dioxide pipeline; and a heat source outlet thereof is connected to the condensate water pipeline and the circulation carbon dioxide pipeline; and
a non-nitrogen inert gaseous diluent pipeline is further included, and the non-nitrogen inert gaseous diluent pipeline is connected to the circulation flue gas pipeline or the circulation main pipeline.

The flue gas heat exchange system further comprises a second heat exchanger located between the first heat exchanger and the condensate water pipeline, that is, a heat source inlet of the second heat exchanger is connected to the heat source outlet of the first heat exchanger;
more preferably, the second heat exchanger comprises at least 2 heat exchange units connected in series; a cold source inlet of the heat exchange unit H2-1 is connected to the fuel pipeline, a cold source outlet thereof is connected to the cold source inlet of the heat exchange unit H1-2 of the first heat exchanger or the burner, a heat source inlet thereof is connected to the heat source outlet of the first heat exchanger, and a heat source outlet thereof is connected to a heat source inlet of heat exchange unit H2-2;
a cold source inlet of the heat exchange unit H2-2 is connected to the oxygen gas pipeline, a cold source outlet thereof is connected to the circulation main pipeline, the heat source inlet thereof is connected to the heat source outlet of the heat exchange unit H2-1, and a heat source outlet thereof is connected to the condensate water pipeline.

Finally, the control system comprises control valves located on the circulation flue gas pipeline, the oxygen gas pipeline, the fuel pipeline, the circulation flue gas pipeline, the oxygen gas pipeline, and fuel pipeline respectively, to control an amount of gas; and a control valve located on the circulation carbon dioxide pipeline to control an amount of carbon dioxide in the circulation carbon dioxide pipeline.

**The method for reducing nitrogen oxide emission of the present application comprises the following steps:**
(1) opening the control valve on the non-nitrogen inert gaseous diluent pipeline to introduce the non-nitrogen inert gaseous diluent, feeding the flue gas from the outlet of the convection section into the circulation main pipeline through the circulation flue gas pipeline under the action of the induced draft fan, and discharging through an exhaust pipeline for replacement until the analysis is qualified (a sample from the exhaust pipeline on the circulation flue gas pipeline near the circulation main pipeline is collected and tested, the nitrogen gas is required to meet the NOx emission requirements, and the oxygen gas and hydrocarbons are required to meet the explosion-proof requirements); then injecting pure oxygen gas into the circulation flue gas pipeline through the oxygen gas pipeline, introducing fuel in the fuel pipeline into the burner of the furnace and performing ignition for combustion when the oxygen gas reaches 10-30%; and after the temperature of the firebox was increased, using the circulation flue gas as a new diluent, gradually reducing the introduction amount of the non-nitrogen inert gaseous diluent until the exhaust amount of flue gas reaches a designed value of the furnace, and then closing the valve of the non-nitrogen inert gaseous diluent pipeline;
(2) introducing a part of the flue gas (a volume ratio of which to the amount of the circulation flue gas is 25-100% , preferably 70-100%) from the flue gas outlet into the heat source inlet of the first heat exchanger and cooling it in the first heat exchanger to obtain a cooled flue gas and a liquid at the heat source outlet of the first heat exchanger, wherein the cooled flue gas is carbon dioxide, and the liquid is liquid water; and feeding a part of the carbon dioxide into the flue gas circulation system through the circulation carbon dioxide pipeline, and introducing the remaining carbon dioxide into the carbon dioxide capture unit, wherein, preferably, a volume ratio of the cooled flue gas fed into the carbon dioxide capture unit to the cooled flue gas from the heat source outlet of the first heat exchanger is 9-21: 100;
(3) directly introducing the remaining flue gas from the flue gas outlet into the circulation flue gas pipeline, and mixing it with the carbon dioxide from the circulation carbon dioxide pipeline, and then feeding the obtained mixture into the circulation main pipeline, wherein the carbon dioxide in the circulation carbon dioxide pipeline is subjected to heat exchange in the heat exchange unit H1-4 of the first heat exchanger before entering the circulation flue gas pipeline; and
(4) preheating oxygen gas by the heat exchange unit H2-2 of the second heat exchanger, introducing the preheated oxygen gas into the heat exchange unit H1-3 of the first heat exchanger for heat exchange, then introducing it into the circulation main pipeline to be mixed with the gas introduced into the circulation main pipeline in step L2, and introducing the obtained mixture into the burner as combustion adjuvant gas; and preheating the fuel by the heat exchange unit H2-1 of the second heat exchanger, introducing the preheated fuel into the heat exchange unit H1-2 of the first heat exchanger for heat exchange, and then introducing it into the burner inlet to complete combustion, thereby achieving low nitrogen oxide emission.

### Example 1

This example provides a method for reducing nitrogen oxide emission in an ethylene cracking furnace, wherein the non-nitrogen inert gaseous diluent is low-pressure superheated steam (at a temperature of 210°C), and the method comprises the following steps:
(1) Firstly, the superheated steam was introduced as the diluent through the non-nitrogen inert gaseous diluent pipeline 1-12, the flue gas from the outlet of the convection section 1-2 of the cracking furnace was fed into the circulation main pipeline 1-9 of the cracking furnace through the circulation flue gas pipeline 1-4 of the cracking furnace under the action of the induced draft fan 1-3 of the cracking furnace, and the resultant was discharged through the exhaust pipeline for replacement until the analysis was qualified (a sample from the exhaust pipeline on the circulation flue gas pipeline near the circulation main pipeline was collected and tested, the nitrogen gas met the NOx emission requirements, and the oxygen gas and hydrocarbons met the explosion-proof requirements).
(2) Pure oxygen gas was injected into the circulation flue gas pipeline through the oxygen gas pipeline 1-11 of the cracking furnace to reach an oxygen gas content of 30%; and the fuel (methane) from the fuel pipeline 1-10 was then introduced into the burner 1-8 of the cracking furnace and was ignited for combustion. After the temperature of the ethylene cracking furnace was increased, the amount of the steam introduced into the non-nitrogen inert gaseous diluent pipeline 1-12 was gradually decreased as the amount of the circulation flue gas was increased. When the amount of the flue gas from the outlet of the convection section reached a designed value of the furnace, the introduction of the superheated steam was stopped.
   A supplementary superheated steam was injected into an upstream pipeline of an air inlet of the circulation fan 1-5 of the cracking furnace after passing through the control valve on the non-nitrogen inert gaseous diluent pipeline 1-12, and was fully mixed with the circulation flue gas, then the obtained mixture was fed into the circulation fan 1-5 of the cracking furnace, wherein the injection amount of the superheated steam was gradually reduced according to the flow rate of the circulation flue gas until no more superheated steam was injected.
(3) All the flue gas from the outlet of the convection section 1-2 of the cracking furnace was fed to the first heat exchanger 1-6 of the cracking furnace, in four heat exchange units connected in series in the first heat exchanger, the flue gas was allowed to exchange heat with the cracking raw material, oxygen gas, fuel, and part of the circulation CO₂ in sequence, to condense all the steam in the flue gas and separate it from the CO₂ in the flue gas.
(4) About 9% of CO₂ separated was captured and recycled, while the remaining CO₂ was fed to the circulation main pipeline 1-9 of the cracking furnace by means of the circulation fan 1-5 of the cracking furnace. The condensed water separated from the first heat exchanger 1-6 of the cracking furnace was fed into the second heat exchanger 1-7 of the cracking furnace to exchange heat with the fuel, so as to preheat the fuel. The preheated fuel was allowed to pass through the heat exchange units of the first heat exchanger for heat exchange, and then was fed into the burner 1-8 of the cracking furnace through the fuel pipeline 1-10 of the cracking furnace.
(5) After being subjected to heat exchange by passing through the heat exchange unit H1-1 of the first heat exchanger, the raw material was fed into the convection section 1-2 of the cracking furnace; the oxygen gas was preheated by the heat exchange unit H2-2 of the second heat exchanger, and was introduced into the heat exchange unit H1-3 of the first heat exchanger for heat exchange, and then was introduced into the circulation main pipeline 1-9, in which it was mixed with the gas in the circulation main pipeline 1-9, and then the obtained mixture was fed into the burner of the cracking furnace as a combustion adjuvant gas; the fuel was preheated by the heat exchange unit H2-1 of the second heat exchanger, and was introduced into the heat exchange unit H1-2 of the first heat exchanger for heat exchange, and then was introduced into the burner 1-8 of the cracking furnace to complete combustion, thereby achieving low nitrogen oxide emission, wherein a ratio of the amount of the required oxygen gas to the amount of the fuel was 4.4/1. The fuel used was methane, and finally, the steam accounted for about 20 wt% in the flue gas from the outlet of the cracking furnace.

Compared with a condition using air as a combustion adjuvant medium, when the superheated steam was used as the non-nitrogen inert gaseous diluent, a total fuel consumption amount may be reduced by about 15%. After calculation, one cracking furnace with a capacity of 150,000 tons/year can save up to 8,000 tons/year of fuel consumption. Since there is no nitrogen gas contained in the combustion adjuvant gas, only a part of the air was sucked in due to negative pressure operation of the firebox, such that the NOx content in the flue gas may be reduced to 10 mg/Nm³ or less.

### Example 2

This example provides a method for reducing nitrogen oxide emission in an ethylene cracking furnace, wherein the non-nitrogen inert gaseous diluent is a mixture of CO₂ and superheated steam (at a temperature of 240°C), and the method comprises the following steps:
(1) Firstly, the mixture of CO₂ and superheated steam was introduced as the diluent through the non-nitrogen inert gaseous diluent pipeline 1-12, the flue gas from the outlet of the convection section 1-2 of the cracking furnace was fed into the circulation main pipeline 1-9 of the cracking furnace through the circulation flue gas pipeline 1-4 of the cracking furnace under the action of the induced draft fan 1-3 of the cracking furnace, and the resultant was discharged through the exhaust pipeline for replacement until the analysis was qualified (a sample from the exhaust pipeline on the circulation flue gas pipeline near the circulation main pipeline was collected and tested, the nitrogen gas met the NOx emission requirements, and the oxygen gas and hydrocarbons met the explosion-proof requirements). Pure oxygen gas was injected into the circulation flue gas pipeline through the oxygen gas pipeline 1-11 of the cracking furnace to reach an oxygen gas content of 10%; and the fuel (a mixture of methane and hydrogen gas, wherein a volume of methane accounts for 50 mol%) was introduced into the burner 1-8 of the cracking furnace and was ignited for combustion. After the temperature of the ethylene cracking furnace was increased, the amount of the CO₂ and superheated steam introduced into the non-nitrogen inert gaseous diluent pipeline 1-12 was gradually decreased as the amount of the circulation flue gas was increased. When the amount of the flue gas from the outlet of the convection section reached a designed value of the furnace, the introduction of the CO₂ and superheated steam was stopped.
   The mixture of CO₂ and superheated steam was injected into an upstream pipeline of an air inlet of a circulation fan 1-5 of the cracking furnace after passing through the control valve on the non-nitrogen inert gaseous diluent pipeline 1-12, and was fully mixed with the circulation flue gas, then the obtained mixture was fed into the circulation fan 1-5 of the cracking furnace, wherein, according to the flow rate of the circulation flue gas, the steam content in the circulation flue gas was gradually reduced to about 20% by controlling the injection amount of the superheated steam and a ratio of the superheated steam to CO₂.
(2) 70% of the flue gas from the outlet of the convection section 1-2 of the cracking furnace was fed to the first heat exchanger 1-6 of the cracking furnace, in four heat exchange units connected in series in the first heat exchanger, the flue gas was allowed to exchange heat with the cracking raw material, oxygen gas, fuel, and part of the circulation CO₂ in sequence, to condense all the steam in the flue gas and separate it from the CO₂ in the flue gas.
(3) About 15% of CO₂ separated was captured and recycled, while the remaining CO₂ was fed to the circulation main pipeline 1-9 of the cracking furnace by means of the circulation fan 1-5 of the cracking furnace. The condensed water separated from the first heat exchanger 1-6 of the cracking furnace was fed into the second heat exchanger 1-7 of the cracking furnace to exchange heat with the fuel, so as to preheat the fuel. The preheated fuel was allowed to pass through the heat exchange units of the first heat exchanger for heat exchange, and then was fed into the burner 1-8 of the cracking furnace through the fuel pipeline 1-10 of the cracking furnace.
(4) After being subjected to heat exchange by passing through the heat exchange unit H1-1 of the first heat exchanger, the raw material was fed into the convection section 1-2 of the cracking furnace; the oxygen gas was preheated by the heat exchange unit H2-2 of the second heat exchanger, and was introduced into the heat exchange unit H1-3 of the first heat exchanger for heat exchange, and then was introduced into the circulation main pipeline 1-9, in which it was mixed with the gas in the circulation main pipeline 1-9, and then the obtained mixture was fed into the burner of the cracking furnace as a combustion adjuvant gas; the fuel was preheated by the heat exchange unit H2-1 of the second heat exchanger, and was introduced into the heat exchange unit H1-2 of the first heat exchanger for heat exchange, and then was introduced into the burner 1-8 of the cracking furnace to complete combustion, thereby achieving low nitrogen oxide emission, wherein a ratio of the amount of the required oxygen gas to the amount of the fuel was 4.8/1. The fuel used was a mixture of methane and hydrogen gas, the methane accounted for 50 mol%.

Compared with a condition using air as a combustion adjuvant medium, when the mixture of the superheated steam and CO₂ was used as the inert gas in the combustion adjuvant medium, a total fuel consumption amount may be reduced by about 4.5%. Since there is no nitrogen gas contained in the combustion adjuvant gas, only a part of the air was sucked in due to negative pressure operation of the firebox, such that the NOx content in the flue gas may be reduced to 10 mg/Nm³ or less.

### Example 3

This example provides a method for reducing nitrogen oxide emission in an ethylene cracking furnace, wherein the non-nitrogen inert gaseous diluent is CO₂, and the method comprises the following steps:
(1) Firstly, the CO₂ was introduced as the diluent through the non-nitrogen inert gaseous diluent pipeline 1-12, the flue gas from the outlet of the convection section 1-2 of the cracking furnace was fed into the circulation main pipeline 1-9 of the cracking furnace through the circulation flue gas pipeline 1-4 of the cracking furnace under the action of the induced draft fan 1-3 of the cracking furnace, and the resultant was discharged through the exhaust pipeline for replacement until the analysis was qualified (a sample from the exhaust pipeline on the circulation flue gas pipeline near the circulation main pipeline was collected and tested, the nitrogen gas met the NOx emission requirements, and the oxygen gas and hydrocarbons met the explosion-proof requirements). Pure oxygen gas was injected into the circulation flue gas pipeline through the oxygen gas pipeline 1-11 of the cracking furnace to reach an oxygen gas content of 20%; and the fuel (a mixture of methane and hydrogen gas, wherein a volume of methane accounts for 60 mol%) was then introduced into the burner 1-8 of the cracking furnace and was ignited for combustion. After the temperature of the ethylene cracking furnace was increased, as the amount of the circulation flue gas was increased, the introduction of the CO₂ was stopped when the amount of the flue gas from the outlet of the convection section reached a designed value of the furnace.
   The CO₂ was injected into an upstream pipeline of an air inlet of a circulation fan 1-5 of the cracking furnace after passing through the control valve on the non-nitrogen inert gaseous diluent pipeline 1-12, and was fully mixed with the circulation flue gas, then the obtained mixture was fed into the circulation fan 1-5 of the cracking furnace.
(2) 50% of the flue gas from the outlet of convection section 1-2 of the cracking furnace was fed to the first heat exchanger 1-6 of the cracking furnace, in four heat exchange units connected in series in the first heat exchanger, the flue gas was allowed to exchange heat with the cracking raw material, oxygen gas, fuel, and part of circulation CO₂ in sequence, to condense all the steam in the flue gas and separate it from the CO₂ in the flue gas.
(3) About 17% of CO₂ separated was captured and recycled, while the remaining CO₂ was fed to the circulation main pipeline 1-9 of the cracking furnace by means of the circulation fan 1-5 of the cracking furnace. The condensed water separated from the first heat exchanger 1-6 of the cracking furnace was fed into the second heat exchanger 1-7 of the cracking furnace to exchange heat with the fuel, so as to preheat the fuel. The preheated fuel was fed into the burner 1-8 of the cracking furnace through the fuel pipeline 1-10 of the cracking furnace.
(4) After being subjected to heat exchange by passing through the heat exchange unit H1-1 of the first heat exchanger, the raw material was fed into the convection section 1-2 of the cracking furnace; the oxygen gas was preheated by the heat exchange unit H2-2 of the second heat exchanger, and was introduced into the heat exchange unit H1-3 of the first heat exchanger for heat exchange, and then was introduced into the circulation main pipeline 1-9, in which it was mixed with the gas in the circulation main pipeline 1-9, and then the obtained mixture was fed into the burner of the cracking furnace as a combustion adjuvant gas; the fuel was preheated by the heat exchange unit H2-1 of the second heat exchanger, and was introduced into the heat exchange unit H1-2 of the first heat exchanger for heat exchange, and then was introduced into the burner 1-8 of the cracking furnace to complete combustion, thereby achieving low nitrogen oxide emission, wherein a ratio of the amount of the required oxygen gas to the amount of the fuel was 4.8/1. The fuel used was a mixture of methane and hydrogen gas, the methane accounted for 60 mol%.

Compared with a condition using air as a combustion adjuvant medium, when the CO₂ was used as the inert gas in the combustion adjuvant medium, a total fuel consumption amount may be reduced by about 1.5%. Since there is no nitrogen gas contained in the combustion adjuvant gas, only a part of the air was sucked in due to negative pressure operation of the firebox, such that the NOx content in the flue gas may be reduced to 10 mg/Nm³ or less.

### Example 4

This example provides a method for reducing nitrogen oxide emission in an refinery heating furnace, wherein the non-nitrogen inert gaseous diluent is superheated steam (at a temperature of 300°C), and the method comprises the following steps:
(1) Firstly, the superheated steam (at a temperature of 300°C) was introduced as the diluent through the non-nitrogen inert gaseous diluent pipeline 2-11, the flue gas from the outlet of the convection section 2-2 of the heating furnace was fed into the circulation main pipeline 2-8 of the heating furnace through the circulation flue gas pipeline 2-3 of the heating furnace under the action of the circulation fan 2-4 of the heating furnace, and the resultant was discharged through the bypass flue 2-12 of heating furnace for replacement until the analysis was qualified (a sample from the exhaust pipeline on the circulation flue gas pipeline near the circulation main pipeline was collected and tested, the nitrogen gas met the NOx emission requirements, and the oxygen gas and hydrocarbons met the explosion-proof requirements). Pure oxygen gas was then injected into the circulation flue gas pipeline through the oxygen gas pipeline 2-10 of the heating furnace to reach an oxygen gas content of 30%; and the fuel (with methane accounting for 95 vol%) was then introduced into the burner of the heating furnace 2-1 and was ignited for temperature elevation. After the temperature of the refinery heating furnace was increased, the amount of the steam introduced into the non-nitrogen inert gaseous diluent pipeline 2-11 was gradually decreased as the amount of the circulation flue gas was increased. When the amount of the flue gas from the outlet of the convection section reached a designed value of the furnace, the introduction of the superheated steam was stopped.
   The superheated steam was injected into an upstream pipeline of an air inlet of the circulation fan 2-4 of the heating furnace after passing through the control valve on the non-nitrogen inert gaseous diluent pipeline 2-11, wherein the injection amount of the superheated steam was gradually reduced according to the flow rate of the circulation CO₂ flue gas until no more superheated steam was injected. Finally, the steam in the flue gas from the outlet of the heating furnace accounted for about 18 wt%.
(2) All the flue gas from the outlet of the convection section 2-2 of the heating furnace was fed to the first heat exchanger 2-5 of the heating furnace, in which the flue gas was allowed to exchange heat with the heating raw material, oxygen gas, fuel, or part of circulation CO₂ in sequence, to condense all the steam in the flue gas and separate it from the CO₂ in the flue gas.
(3) About 9% of CO₂ separated was captured and recycled, while the remaining CO₂ was fed to the circulation main pipeline 2-8 of the heating furnace by means of the circulation fan 2-4 of the heating furnace. The condensed water separated from the first heat exchanger 2-5 of the heating furnace was fed into the second heat exchanger 2-6 of the heating furnace to exchange heat with the fuel, so as to preheat the fuel. The preheated fuel was allowed to pass through the heat exchange units of the first heat exchanger for heat exchange, and then was fed into the burner 2-7 of the heating furnace through the fuel pipeline 2-9 of the heating furnace.
(4) After being subjected to heat exchange by passing through the heat exchange unit H1-1 of the first heat exchanger, the raw material was fed into the convection section 2-2 of the heating furnace; the oxygen gas was preheated by the heat exchange unit H2-2 of the second heat exchanger, and was introduced into the heat exchange unit H1-3 of the first heat exchanger for heat exchange, and then was introduced into the circulation main pipeline 2-8, in which it was mixed with the gas in the circulation main pipeline 2-8, and then the obtained mixture was fed into the burner of the heating furnace as a combustion adjuvant gas; the fuel was preheated by the heat exchange unit H2-1 of the second heat exchanger, and was introduced into the heat exchange unit H1-2 of the first heat exchanger for heat exchange, and then was introduced into the burner 2-7 of the heating furnace to complete combustion, thereby achieving low nitrogen oxide emission, wherein a ratio of the amount of the required oxygen gas to the amount of the fuel was 4.4/1. The fuel used was methane, and finally, the steam accounted for about 28 wt% in the flue gas from the outlet of the heating furnace.

Compared with a condition using air as a combustion adjuvant medium, when the superheated steam was used as the non-nitrogen inert gaseous diluent, a total fuel consumption amount may be reduced by about 15%. Since there is no nitrogen gas contained in the combustion adjuvant gas, only a part of the air was sucked in due to negative pressure operation of the firebox, such that the NOx content in the flue gas may be reduced to 10 mg/Nm³ or less.

### Example 5

This example provides a method for reducing nitrogen oxide emission in an ethylene cracking furnace, wherein the non-nitrogen inert gaseous diluent is low-pressure superheated steam (at a temperature of 250°C), and the method comprises the following steps:
(1) Air was directly introduced as the diluent through the exhaust pipeline, the flue gas from the outlet of the convection section 1-2 of the cracking furnace was fed into the circulation main pipeline 1-9 of the cracking furnace through the circulation flue gas pipeline 1-4 of the cracking furnace under the action of the induced draft fan 1-3 of the cracking furnace, and the resultant was then discharged through the exhaust pipeline for replacement until the analysis was qualified (a sample from the exhaust pipeline on the circulation flue gas pipeline near the circulation main pipeline was collected and tested, the oxygen gas and hydrocarbons met the explosion-proof requirements).
(2) The fuel (methane) from the fuel pipeline 1-10 was then introduced into the burner 1-8 of the cracking furnace and was ignited for combustion. When the temperature of the ethylene cracking furnace was increased to the temperature of the diluent (250°C) or higher, the low-pressure superheated steam was introduced as a diluent through the non-nitrogen inert gaseous diluent pipeline 1-12. The flue gas from the outlet of the convection section 1-2 of the cracking furnace was fed to the circulation main pipeline 1-9 of the cracking furnace through the circulation flue gas pipeline 1-4 of the cracking furnace under the action of the induced draft fan 1-3 of the cracking furnace, and then the resultant was discharged through the exhaust pipeline for replacement. The exhaust pipeline that introduced air was gradually closed while the flow rate of the diluent was increased. The oxygen gas pipeline 1-11 was opened to introduce oxygen gas, until the analysis was qualified (the nitrogen oxide content met local environmental requirements), and the introduction of the superheated steam was stopped.
   A supplementary superheated steam was injected into an upstream pipeline of an air inlet of a circulation fan 1-5 of the cracking furnace after passing through the control valve on the non-nitrogen inert gaseous diluent pipeline 1-12, and was fully mixed with the circulation flue gas, then the obtained mixture was fed into the circulation fan 1-5 of the cracking furnace, until no more superheated steam was injected.
(3) All the flue gas from the outlet of convection section 1-2 of the cracking furnace was fed to the first heat exchanger 1-6 of the cracking furnace, in one heat exchange unit of the first heat exchanger, the flue gas was allowed to exchange heat with the cracking raw material.
(4) After being subjected to heat exchange by passing through the heat exchange unit H1-1 of the first heat exchanger, the raw material was fed into the convection section 1-2 of the cracking furnace; the oxygen gas was introduced into the circulation main pipeline 1-9, in which it was mixed with the gas in the circulation main pipeline 1-9, and then the obtained mixture was fed into the burner of the cracking furnace as a combustion adjuvant gas; wherein a ratio of the amount of the required oxygen gas to the amount of the fuel was 4.4/1. The fuel used was methane, and finally, the steam accounted for about 16 wt% in the flue gas from the outlet of the cracking furnace.

Compared with a condition using air as a combustion adjuvant medium, when the superheated steam was used as the non-nitrogen inert gaseous diluent, a total fuel consumption amount may be reduced by about 1.5%. Since there is no nitrogen gas contained in the combustion adjuvant gas, only a part of the air was sucked in during the initial replacement stage and due to negative pressure operation of the firebox, such that the NOx content in the flue gas may be reduced to 20 mg/Nm³ or less.

### Comparative Example 1:

This comparative example provides a method for reducing nitrogen oxide emission in an refinery heating furnace, wherein the non-nitrogen inert gaseous diluent is superheated steam (at a temperature of 180°C), and the method comprises the following steps:
(1) The superheated steam (at a temperature of 180°C) was introduced as the diluent through the non-nitrogen inert gaseous diluent pipeline 2-11, the flue gas from the outlet of the convection section 2-2 of the heating furnace was fed into the circulation main pipeline 2-8 of the heating furnace through the circulation flue gas pipeline 2-3 of the heating furnace under the action of the circulation fan 2-4 of the heating furnace, and the resultant was discharged through the bypass flue 2-12 of heating furnace for replacement until the analysis was qualified (a sample from the exhaust pipeline on the circulation flue gas pipeline near the circulation main pipeline was collected and tested, the oxygen gas and hydrocarbons met the explosion-proof requirements, and the nitrogen gas met the NOx emission requirements). Pure oxygen gas was then injected into the circulation flue gas pipeline through the oxygen gas pipeline 2-10 of the heating furnace to reach an oxygen gas content of 15%; and the fuel (hydrogen gas) was then introduced into the burner of the heating furnace 2-1 and was ignited for temperature elevation. After the temperature of the refinery heating furnace was increased, the amount of the steam introduced into the non-nitrogen inert gaseous diluent pipeline 2-11 was gradually decreased as the amount of the circulation flue gas was increased. When the amount of the flue gas from the outlet of the convection section reached a designed value of the furnace, the introduction of the superheated steam was stopped.
   The superheated steam was injected into an upstream pipeline of an air inlet of a circulation fan 2-4 of the heating furnace after passing through the control valve on the non-nitrogen inert gaseous diluent pipeline 2-11, and was fully mixed with the circulation flue gas and then the obtained mixture was fed into the circulation fan 2-4 of the heating furnace, wherein the injection amount of the superheated steam was gradually reduced according to the flow rate of the circulation flue gas until no more superheated steam was injected.
(2) 84% of the flue gas from the outlet of convection section 2-2 of the heating furnace was fed to the circulation main pipeline 2-8 of the heating furnace, and 16% of the flue gas was discharged out of the system.
(3) The oxygen gas was introduced into the circulation main pipeline 2-8, in which it was mixed with the gas in the circulation main pipeline 2-8, and then the obtained mixture was fed into the burner of the heating furnace as a combustion adjuvant gas; and the fuel was introduced into the burner 2-7 of the heating furnace to complete combustion, thereby achieving low nitrogen oxide emission and zero carbon emission, wherein a ratio of the amount of the required oxygen gas to the amount of the fuel was 8.8/1. The fuel used was hydrogen gas, and finally, the steam accounted for about 100 wt% in the flue gas from the outlet of the heating furnace.

Compared with a condition using air as a combustion adjuvant medium, when the superheated steam was used as the non-nitrogen inert gaseous diluent, since there is no nitrogen gas contained in the combustion adjuvant gas, only a part of the air was sucked in due to negative pressure operation of the firebox, such that the NOx content in the flue gas may be reduced to 10 mg/Nm³ or less.

It should be noted that, the above examples are only used to explain the present invention, but will not constitute any limitation on the present invention. The present invention has been described with reference to typical examples, but it should be understood that, the words used therein are descriptive and explanatory vocabularies, rather than restrictive vocabularies. Modifications may be made to the present invention as regulated within the scope of the claims of the present invention, and revisions may be made to the present invention without departing from the scope of the present invention. Although the present invention described herein relates to specific methods, materials, and examples, it does not mean that the present invention is limited to the specific examples disclosed therein. On the contrary, the present invention may be extended to all the other methods and applications with the same functionality.

## Claims

1. A method for reducing nitrogen oxide emission, **characterized by** comprising reducing N₂ content in a system by the following manners:
I. introducing a non-nitrogen inert gaseous diluent to replace gas inside a furnace;
II. mixing a non-nitrogen inert gaseous diluent and oxygen gas, introducing the mixed gas into the firebox to be mixed with a fuel, and then igniting the mixture to initiate combustion; and/or
III. cooling at least a part of flue gas from the flue gas outlet to obtain a cooled flue gas, mixing a part of the cooled flue gas and remaining flue gas from the flue gas outlet with oxygen gas, or mixing all the flue gas from the flue gas outlet with oxygen gas, then introducing the obtained mixture into the firebox to be mixed with the fuel for combustion;
wherein, the non-nitrogen inert gaseous diluent is selected from one or two of CO₂ gas and superheated steam.

2. The method for reducing nitrogen oxide emission of claim 1, **characterized in that**, in manner II, a condition for ignition comprises: a volume ratio of the oxygen gas in the mixed gas of the non-nitrogen inert gaseous diluent and the oxygen gas reaches 10-30%; and/or
in manner III, a volume ratio of the flue gas which is subjected to cooling to all the flue gas is 25-100%; and/or
a temperature of the superheated steam as the non-nitrogen inert gaseous diluent is 120-300°C.

3. The method for reducing nitrogen oxide emission of claim 1 or 2, **characterized in that**, the manner III comprises:
feeding at least a part of the flue gas from the flue gas outlet into a heat source inlet of a first heat exchanger and cooling it in the first heat exchanger, to obtain the cooled flue gas at a heat source outlet of the first heat exchanger; and feeding a part of the cooled flue gas and the remaining flue gas from the flue gas outlet into the firebox;
preferably, the cooled flue gas is carbon dioxide, and more preferably, a remaining cooled flue gas is fed to a carbon dioxide capture unit for collection.

4. The method for reducing nitrogen oxide emission of claim 3, **characterized in that**, a volume ratio of the cooled flue gas fed into the carbon dioxide capture unit to the cooled flue gas from the heat source outlet of the first heat exchanger is 9-21:100; and/or
the first heat exchanger comprises one or more heat exchange units connected in series, with a heat fluid being the flue gas, and with a cold fluid being independently of O₂, the fuel, the cooled flue gas to be fed into the burner or a raw material to be treated, the first heat exchanger is used for heating the O₂, the fuel, the cooled flue gas to be fed into the burner or the raw material to be treated; and/or
a second heat exchanger is further included, with a heat fluid being a liquid at the heat source outlet of the first heat exchanger, and a cold fluid being independently O₂, the fuel or a raw material, and the second heat exchanger is used for preheating the O₂, the fuel or the raw material;
preferably, the O₂ and/or the fuel to be fed into the burner are firstly fed into a cold source inlet of the second heat exchanger for preheating; and the preheated O₂ and/or fuel are then fed into cold source inlets of individual heat exchange units of the first heat exchanger for heating.

5. The method for reducing nitrogen oxide emission of any one of claims 1-4, **characterized by** comprising the following steps:
optionally, (1) filling the non-nitrogen inert gaseous diluent into the firebox through a non-nitrogen inert gaseous diluent pipeline, and discharging it through an outlet of a convection section and/or an exhaust pipeline on a circulation flue gas pipeline to replace gas inside the firebox;
(2) injecting oxygen gas into a circulation main pipeline or the circulation flue gas pipeline through an oxygen gas pipeline; injecting the non-nitrogen inert gaseous diluent into the circulation main pipeline or the circulation flue gas pipeline through the non-nitrogen inert gaseous diluent pipeline; allowing the flue gas from the flue gas outlet to pass through the circulation flue gas pipeline and the circulation main pipeline in sequence, then introducing it into the firebox; after a predetermined oxygen gas content is reached, introducing the fuel into the burner through a fuel pipeline and igniting the mixture to initiate combustion; and stopping the introduction of the non-nitrogen inert gaseous diluents until an amount of circulation flue gas reaches a designed minimum required value; and
(3) cooling at least a part of the flue gas from the flue gas outlet to obtain the cooled flue gas, mixing in the circulation main pipeline a part of the cooled flue gas and the remaining flue gas from the flue gas outlet with oxygen gas from the oxygen gas pipeline, introducing the obtained mixture into the firebox, then introducing the fuel into the burner through the fuel pipeline, to allow continuous combustion.

6. The method for reducing nitrogen oxide emission of any one of claims 1-4, **characterized in that** manner I further comprises replacing gas inside the furnace by adopting the following steps:
S1. introducing air through an air inlet located on the circulation flue gas pipeline or the circulation main pipeline, and after analysis in the firebox is qualified, introducing a fuel for ignition;
S2. when a temperature of the flue gas leaving a radiation section is higher than a temperature of the non-nitrogen inert gaseous diluent, introducing the non-nitrogen inert gaseous diluent to replace gas inside the furnace; and gradually stopping the introduction of the air while increasing a flow rate of the diluent.

7. The method for reducing nitrogen oxide emission of claim 5, **characterized in that**, an amount of the circulation flue gas reaches the designed minimum required value, which is an amount of flue gas as a diluent required during normal operation of the furnace at a lowest load; and/or
the remaining cooled flue gas of step (3) is fed to a carbon dioxide capture unit.

8. A combustion system with low nitrogen oxide emission, **characterized in that**, the combustion system comprises a furnace, a flue gas circulation system, an oxygen gas pipeline, a fuel pipeline, and a control system;
the furnace is sequentially provided with a radiation section and a convection section; the radiation section is provided therein with a burner; the flue gas outlet is used for emission of flue gas after combustion;
the flue gas circulation system comprises a circulation flue gas pipeline and a circulation main pipeline; two ends of the circulation flue gas pipeline are respectively connected to the flue gas outlet and the circulation main pipeline respectively, and are used for feeding the flue gas from the flue gas outlet, i.e. a circulation flue gas, into the circulation main pipeline, to achieve circulation of the flue gas;
the oxygen gas pipeline is connected to the circulation main pipeline or the circulation flue gas pipeline, and is used for feeding oxygen gas into the circulation main pipeline;
the circulation main pipeline is connected to an burner and is used for feeding a mixed gas of the circulation flue gas or a non-nitrogen inert gaseous diluent and oxygen gas into the firebox;
the fuel pipeline is connected to the burner, and is used for feeding a fuel into the burner;
a non-nitrogen inert gaseous diluent pipeline is further included, and the non-nitrogen inert gaseous diluent pipeline is connected to the circulation flue gas pipeline or the circulation main pipeline.

9. The combustion system of claim 8, **characterized in that**, the flue gas circulation system further comprises a circulation carbon dioxide pipeline; the circulation carbon dioxide pipeline is connected to the outlet of the convection section and the circulation flue gas pipeline; the combustion system further comprises a flue gas heat exchange system located between the circulation carbon dioxide pipeline and the outlet of the convection section, wherein the flue gas heat exchange system comprises a first heat exchanger; and/or
the outlet of the convection section is connected to a heat source inlet of the first heat exchanger, a heat source outlet of the first heat exchanger is connected to an inlet of the circulation carbon dioxide pipeline; an outlet of the circulation carbon dioxide pipeline is connected to the circulation flue gas pipeline or the circulation main pipeline and is used for feeding a cooled flue gas into the circulation flue gas pipeline or the circulation main pipeline, and in turn into the firebox, and an inlet of a carbon dioxide capture unit is connected to the heat source outlet of the first heat exchanger.

10. The combustion system of claim 9, **characterized in that**, the first heat exchanger comprises one or more heat exchange units connected in series; wherein heat fluid is the flue gas, and cold fluid is O₂, a fuel, a circulation CO₂ or a raw material to be treated, which are independently to be fed into the burner, and the first heat exchanger is used for heating the O₂, the fuel, the circulation CO₂ or the raw material to be treated; preferably, the first heat exchanger comprises 2-4 heat exchange units connected in series, wherein a heat source inlet of a first heat exchange unit is connected to the outlet of the convection section; a heat source outlet of each heat exchange unit is connected to a condensate water pipeline, and a heat source outlet of the last heat exchanger is connected to the circulation carbon dioxide pipeline;
more preferably, the first heat exchanger comprises a heat exchange unit H1-1, a heat exchange unit H1-2, a heat exchange unit H1-3, and a heat exchange unit H1-4 connected in series, wherein a cold source inlet of the heat exchange unit H1-1 is connected to a raw material pipeline, a cold source outlet thereof is connected to the convection section of the furnace, a heat source inlet thereof is connected to the outlet of the convection section, and a heat source outlet thereof is connected to the condensate water pipeline and a heat source inlet of heat exchange unit H1-2;
a cold source inlet of the heat exchange unit H1-2 is connected to the fuel pipeline, a cold source outlet thereof is connected to the burner, and a heat source outlet thereof is connected to the condensate water pipeline and a heat source inlet of heat exchange unit H1-3;
a cold source inlet of the heat exchange unit H1-3 is connected to the oxygen gas pipeline, a cold source outlet thereof is connected to the circulation main pipeline, and a heat source outlet thereof is connected to the condensate water pipeline and a heat source inlet of heat exchange unit H1-4; and
a cold source inlet of the heat exchange unit H1-4 is connected to a partial circulation carbon dioxide pipeline, a cold source outlet thereof is connected to the circulation carbon dioxide pipeline, for heating gas in the partial circulation carbon dioxide pipeline; and a heat source outlet thereof is connected to the condensate water pipeline and the circulation carbon dioxide pipeline.

11. The combustion system of claim 9 or 10, **characterized in that**, the flue gas heat exchange system further comprises a second heat exchanger located between the first heat exchanger and the condensate water pipeline, that is, a heat source inlet of the second heat exchanger is connected to the heat source outlet of the first heat exchanger; and cold fluid is independently O₂, the fuel or the raw material, and the second heat exchanger is used for preheating O₂, the fuel or the raw material; and/or
the second heat exchanger comprises at least 2 heat exchange units connected in series;
preferably, the O₂ and/or fuel to be fed into the burner is first fed into a cold source inlet of the second heat exchanger for preheating; and the preheated O₂ and/or the fuel and the circulation CO₂ and/or the raw material, enter the cold source inlets of individual heat exchange units of the first heat exchanger respectively, to exchange heat with the circulation flue gas;
preferably, the second heat exchanger comprises a heat exchange unit H2-1 and a heat exchange unit H2-2, a cold source inlet of the heat exchange unit H2-1 is connected to the fuel pipeline, a cold source outlet thereof is connected to the cold source inlet of the heat exchange unit H1-2 of the first heat exchanger or the burner, a heat source inlet thereof is connected to the heat source outlet of the first heat exchanger, and a heat source outlet thereof is connected to a heat source inlet of heat exchange unit H2-2; and/or
a cold source inlet of the heat exchange unit H2-2 is connected to the oxygen gas pipeline, a cold source outlet thereof is connected to the circulation main pipeline, and a heat source outlet thereof is connected to the condensate water pipeline.

12. The combustion system with low nitrogen oxide emission of any one of claims 8-11, **characterized in that**, the control system comprises: control valves located on the circulation flue gas pipeline, the diluent pipeline, the oxygen gas pipeline, the fuel pipeline respectively, to control an amount of gas; and a control valve located on the circulation carbon dioxide pipeline to control an amount of carbon dioxide in the circulation carbon dioxide pipeline.

13. The method for reducing nitrogen oxide emission of any one of claims 1-7 or the combustion system with low nitrogen oxide emission of any one of claims 8-12, **characterized in that**, the furnace is a fire heating furnace, preferably, a refinery heating furnace or an ethylene cracking furnace.
